# EUROPEAN PATENT SPECIFICATION

(11) **EP 1 412 470 B1**
(45) Date of publication and mention of the grant of the patent: **03.11.2010**
(21) Application number: 02758725.2
(22) Date of filing: 24.07.2002
(51) Int. Cl.: C12Q 1/04

(54) **AUTOMATED PROCESS FOR DETECTING PATHOGENIC ORGANISMS IN WATER**
AUTOMATISCHES VERFAHREN ZUM NACHWEIS PATHOGENER ORGANISMEN IN WASSER
PROCEDE AUTOMATISE DESTINE A DETECTER DES ORGANISMES PATHOGENES DANS L'EAU

(30) Priority: 24.07.2001 FR 0109841; 24.07.2001 US 307171 P
(43) Date of publication of application: 28.04.2004
(73) Proprietor: SOCIETE D'AMENAGEMENT URBAIN ET RURAL, 78280 Guyancourt (FR)
(72) Inventor: HUAU, Marie-Christine, F-75016 Paris (FR); SCHALKHAMMER, Thomas, Casten 105 (AT)
(74) Representative: Warcoin, Jacques
(86) International application number: PCT/IB2002/003424
(87) International publication number: WO 2003/010278

(56) References cited:
- WO-A-00/78455
- WO-A-01/46477
- WO-A-98/42874
- US-A- 5 298 392
- US-B1- 6 187 530
- ATLAS R M ET AL: "DETECTING BACTERIAL PATHOGENS IN ENVIRONMENTAL WATER SAMPLES BY USING PCR AND GENE PROBES" PCR PROTOCOLS GUIDE TO METHODS AND APPLICATIONS, SAN DIEGO, ACADEMIC PRESS, US, 1989, pages 399-406, XP000363601

## Description

The present invention relates to the general technical domain of the detection of pathogenic organisms in water.

In particular, the present invention relates to an entirely automated process for detecting the presence of pathogenic organisms in a water sample. The invention also relates to a device for detecting the presence of pathogenic organisms in a water sample, for carrying out this process.

The presence of pathogenic agents in water supply networks, such as drinking water or industrial water networks, engenders real risks to the health of human beings. Water supply networks thus, mostly, contain pathogenic organisms, such as viruses, bacteria, protozoa, fungi, amoebae or worms, which are capable of causing various diseases in humans. Among these diseases, mention may be made, inter alia, of cholera, typhoid fever, diarrhoea, dysentery, legionellosis or gastroenteritis.

Among the pathogenic organisms responsible for such diseases, mention may be made more particularly of the Cryptosporidium (parvum) and Giardia Lamblia protozoa both of which originate from human and animal fecal waste and which are a major cause of gastroenteric diseases, the Toxoplasma gondii and Naegleria fowleri protozoa which also originate from human and animal fecal waste and which cause various serious diseases, Cyclospora which cause diarrhoea, and coliform bacteria, in particular Escherichia coli, which are found throughout the biosphere and the detection of which is important since they are considered not to be typical pathogenic agents, but to be organisms which indicate water contamination, as they cause diseases only at very high concentrations. In the context of the present invention, for the purpose of simplification, all organisms which indicate water contamination and all pathogenic organisms actually responsible for diseases in humans will be included in term "pathogenic organisms". Among pathogenic organisms responsible for diseases, mention may also be made of the bacteria Salmonella, including the serotypes S. enteridis, S. enterica, S. typhi and S. paratyphi, which are the cause of 70% of the bacterial diseases of food origin, in particular typhoid fever and paratyphoid fever, and which are the cause of a large number of deaths, Heliobacter pylori, which is an organism linked to the cause of at least 75% of all stomach ulcers and to two types of stomach cancer, and which is also the cause of a large number of deaths, and Legionella, which is found in natural areas and also in water heating systems, which causes Legionnaires' disease, and also viruses of human and animal origin, and in particular hepatitis A viruses, viruses of the Norwalk type, rotaviruses, adenoviruses, enteroviruses and rheoviruses. Norwalk viruses in particular cause sporadic and epidemic gastrointestinal diseases with diarrhoea. Approximately 200 000 cases thereof per year are seen in the United States.

Given the presence of various pathogenic agents in all water supply networks, the latter should be controlled and possibly disinfected before the water which comes from these networks can be considered to be drinking water. With regard to waste water or cooling water supply networks, and also energy production networks, in particular nuclear energy production networks, it is sometimes necessary to analyse the water and even to disinfect it before it can be reused or discarded into the surrounding environment.

The detection of the presence of pathogenic organisms, in particular the detection of slowly growing highly pathogenic organisms, which are the most difficult to detect, thus represents a major challenge in the spheres of water treatment, of water distribution and of the quality control of water. The conventional techniques for detecting pathogenic agents, developed to date, have various drawbacks: they are not used continuously or in real time, they have a relatively low sensitivity with respect to pathogenic organisms and they prove to be long and expensive in terms of material and personnel.

A first known technique uses the counting of microorganisms which develop after culturing the sample on various selective nutrient media. This technique is simple but has considerable risks of error and of artefact (poor specificity of morphological criteria, absence of development of slowly-growing microorganisms, exponential growth on the nutrient medium of bacterial strains which are in reality in the lag phase of growth in the sample). Its response time is, in addition, generally longer than 24 hours.

A second technique, using the measurement of one or more enzymatic activity/activities enables the rapid quantification of populations of living microorganisms (microorganisms which can be cultured and/or microorganisms which are in viable form but which cannot be cultured) (WO 90/100983). This technique in particular allows a set of populations to be monitored, but does not enable a very fine degree of specificity to be obtained.

A third technique, using immunological probes, has a response time which is frequently longer than 24 hours, and often lacks sensitivity and specificity (artefacts due to cross reactions may be observed).

US6,187,530 discloses an aquatic auto-sampler to record the presence of microorganisms in water samples. Collected water is passed through a filter disk and the filtrate passed to chemical sensors downstream. Particles collected on the filter disk can be subjected to one or more reagents to apply molecular probes that label specific species or cause cell lysis in situ. After unloading the filter disk, cells are examined by electron microscopy.

Other recent techniques use specific oligonucleotide probes which are generally labelled so as to allow their detection after hybridization to their targets. Two main types of oligonucleotide probe have been developed: probes with DNAs (or the mRNAs which correspond to them) as the target, and probes with rRNAs (ribosomal RNAs) as the target. However, DNA or mRNA probes, although potentially very specific, have the drawback of being relatively insensitive due to the low number of DNA (or mRNA) copies per microbial cell.

A need thus exists to develop a process and a device for detecting the presence of pathogenic organisms in a water sample, said process and device not having the drawbacks of the techniques of the prior art and thus making it possible to carry out the on-line detection of several pathogenic microorganisms in water in a very short period of time (less than 6 hours), less expensively and with high sensitivity and specificity, making it possible to get a detection limit of 1 organism in 100 ml of water sample analysed and thus to respect the standards imposed on drinking water quality.

The present invention satisfies this need. The Applicant has thus discovered, surprisingly, that this could be carried out with the aid of a process and a device, controlled entirely automatically, which can be used on an industrial scale, preferably taking as a basis the technique of amplifying DNA or RNA using a polymerase chain reaction (PCR), or a similar technique.

The process according to the present invention is thus entirely automated and contains no manual step. It is based on the detection of DNA or RNA sequences specific for certain pathogenic microorganisms. The automation of certain single steps has already been described in the prior art, but the automation of all of the sequential steps of the process according to the present invention has never been envisaged until now. Thus, the extraction on a solid support and the purification of nucleic acids present in a liquid sample have already been automated and have been described in patent applications WO 00/75623 and WO 97/10331. Similarly, the automation of nucleic acid amplification carried out by polymerase chain reaction (PCR), and also the detection of organisms by fluorescence, have already been envisaged in patent application WO 00/33962. However, the problems encountered in carrying out the automation of the five sequential steps of the process according to the present invention have thus never been overcome until now.

A subject of the present invention is thus a process for detecting the presence of pathogenic organisms in a water sample, comprising the succession of the steps as defined in claim 1.

The water analysed in the process for detecting the presence of pathogenic organisms, may be taken from supply networks, such as drinking water networks, industrial or waste water networks, cooling water supply networks, or energy production networks, in particular nuclear energy production networks.

The pathogenic organisms detected are preferably bacteria, viruses, fungi and/or protozoa.

The starting volume of the water sample is preferably of more than 50 milliters, preferably from 100 ml to 10 litres, more preferably from 2 to 3 litres.

According to the present invention, nucleic acid amplification is necessary in the detection process in order to be able to detect a minimum of one pathogenic agent, and preferably from 1 to 5 pathogenic agents, in the water sample analysed.

The nucleic acid amplification may be carried out by a real-time polymerase chain reaction (PCR). PCR is a commonly used amplification technique. The PCR technique (Rolfs et al., 1991), requires the choice of pairs of oligonucleotide primers bordering the fragment which must be amplified. The general principles and the conditions for carrying out nucleic acid amplification by PCR are well known to those skilled in the art and in particular are described in US patents 4,683,202; 4,683,195 and 4,965,188.

Other similar techniques for amplifying nucleic acids (DNA and/or RNA) can advantageously be used as an alternative to PCR (PCR-like) using pairs of primers of nucleotide sequences. The term "PCR-like" is intended to denote all methods using direct or indirect reproductions of nucleic acid sequences, or in which the labelling systems have been amplified, these techniques of course being known to those skilled in the art. Mention may, in particular, be made of the SDA (Strand Displacement Amplification) technique (Walker et al., 1992), the TAS (Transcription-based Amplification System) technique described by Kwoh et al. (1989), the 3SR (Self-Sustained Sequence Replication) technique described by Guatelli et al. (1990), the NASBA (Nucleic Acid Sequence Based Amplification) technique described by Kievitis et al. (1991), the TMA (Transcription Mediated Amplification) technique, the LCR (Ligase Chain Reaction) technique described by Landegren et al. (1988), the RCR (Repair Chain Reaction) technique described by Segev (1992), the CPR (Cycling Probe Reaction) technique described by Duck et *al.* (1990) and the Q-beta-replicase amplification technique described by Miele et al. (1983). Some of these techniques have since been improved. Thus, according to the present invention, specific detection of pathogenic agents can be carried out using such techniques for amplifying nucleic acids at high speed.

In comparing the technique of amplification by PCR or by a similar technique, such as NASBA, with conventional techniques for detecting pathogenic agents, such as cell culture, the Applicant has discovered that it is possible to reduce the length of time required for the assay from a few weeks or a few days to a few hours. In addition, PCR amplification or a similar technique is easy to carry out and the initial costs, like the subsequent costs for carrying out the PCR, prove to be considerably lower than the costs generated for carrying out cell culture techniques. In addition, a PCR can be used to identify a specific form of the pathogenic agents which are found in the water. It can thus be used to detect the infectious state of an organism, proving the presence or absence of DNA or of RNA specific for the pathogenic agent. The PCR amplification according to the invention also makes it possible to obtain a process with high sensitivity, advantageously greater than 1 picogramme, and even more advantageously greater than a few femtogrammes of nucleic acids.

The Applicant has also discovered that the use of PCR amplification (or a similar technique) followed by in situ specific real-time detection, for instance by fluorescence, produces analytical data relating to the detection and to the identification of the organisms more rapidly and more accurately than any other detection system based on biochips. Biochips make it possible to detect a much greater number of pathogenic species than that detected using on-line PCR, but, on the basis of known pathogenic agents (which can be more effectively tested as groups), the real-time PCR is much more effective and gives a better specificity than standard hybridized biochips. In addition, chip hybridization, which allows the microorganisms to be brought into contact with a DNA and/or RNA probe, takes several hours, and even often includes overnight incubation, which is significantly longer than the real-time amplification of the DNA or of the RNA.

A large variety of primers can be used to carry out the nucleic acid amplification, preferably by PCR, according to the present invention, some of these primers already being available in databases or in the literature, certain others having already been developed for specific organisms.

A nested PCR may be used, by dividing the PCR into two steps, in order to obtain maximum selectivity with respect to the pathogenic agents whose detection in the water is being sought. The first PCR is carried out with the entire sample (typically several tubes of 100 - 200 microlitres with only a few DNA molecules) using a set of degenerate primers and employing a standard thermocycler machine. After amplification for a few cycles (for example from 10 to 15 cycles) at high temperature (75 to 100°C), the fluid which then contains at least several thousands of copies of each DNA can be divided into a certain number of vials in a second PCR machine, using a means of automatic sampling and distribution such as a robot. A second set of primers is then added to each vial and a second nucleic acid amplification is then carried out using on-line detection, for example by fluorescence. It will thus be possible to use the first amplification to amplify nucleic acids of groups of pathogenic agents using a set of universal (degenerate) primers, while the second amplification will make it possible to determine accurately the species present in the sample analysed, using a set of primers which are specific to the pathogenic agents whose detection is being sought. In this particular embodiment of the present invention, detection is carried out while carrying out the second PCR whereas no detection is carried out while carrying out the first PCR which only amplifies nucleic acids.

While in the medical domain the analysis of blood samples of a few microlitres to a few millilitres is involved, in research and control relating to the environment, samples having volumes which can range up to several tens of litres are usually analysed. However, the direct techniques for breaking open the cells of the organisms cannot be carried out on such large volumes, and the pathogenic organisms, whose detection is being sought according to the process of the present invention, must be concentrated down to volumes of less than 100 ml before being able to be given the remainder of the treatment. In addition, environmental standards impose that an organism must be proved to be absent in 100 ml of water sample analysed. The difficulty in concentrating pathogenic organisms derived from samples of the order of a litre of water is losing as few organisms as possible while at the same time reducing the volume of the sample down to a volume of about one hundred ml.

According to the present invention, the concentrating of the pathogenic organisms is carried out by precipitation, coprecipitation or precipitation by inclusion. Such a concentrating of organisms starting from sample volumes of about one litre thus makes it possible to have a highly sensitive process and to respect the standards imposed on water quality. Specifically, the greater the volume of a sample, the greater the sensitivity and the more accurate the analytical data. In addition, the detection process according to the present invention makes it possible to detect the presence of less than 10 organisms in initial sample volumes of 100 ml to 10 litres, preferably 2 to 3 litres. Furthermore, although the instruments of the competition use either filtration or capture by magnetic beads as a first concentration step, precipitation by inclusion is more effective in terms of trapping a wide variety of organisms and more economical, and allows highly contaminated samples to be treated. According to the present invention, the concentrating of the pathogenic organisms includes the automatic sampling of water samples analysed.

Even more advantageously, the concentrating of the pathogenic organisms may be carried out in the presence of divalent metal and carbonate ions advantageously chosen from the group consisting of calcium carbonate, strontium carbonate and barium carbonate. The reaction medium is brought to a relatively high temperature, preferably of about 40 to 60°C, so as to allow the rapid formation, in a few minutes, of a precipitate containing various pathogenic agents, simply by settling out and without additional gravitational force. In order to obtain efficient settling out, the reagents such as the divalent carbonate ions are not added to the reaction medium all at once, but are added step by step. After formation of the precipitate, onto which the various pathogenic agents of the water sample adsorb or are trapped within , the supernatant liquid is taken off and the precipitate is dissolved in an acid, such as formic acid. The reaction medium containing the dissolved precipitate is then neutralized with a buffer agent.

Advantageously, the step for breaking open the cells, which follows that for concentrating the pathogenic organisms, may be carried out by grinding on agitated beads. For the purposes of the present invention, the expression "breaking open the cells" is intended to mean rupture or lysis of the cells from the pathogenic organisms, intended to release the content, and in particular the nucleic acids, of these cells. The operating conditions for this step are adjusted so as to avoid any modification of the nucleic acids. The step for breaking open the cells is carried out using analysed sample volumes of about 10 to 100 ml, preferably of 50 ml, and has been designed to preferably take place in a semi-continuous system using beads agitated by a strong source of energy so as to rupture the cells from the organisms by means of direct collisions and impacts. The particles for the splitting open, are retained in the splitting chamber (cracking chamber), whereas the cells ruptured subsequent to the collisions generated by the particles, and the nucleic acids thus released from the cells, are set free from the cracking chamber, thus enabling the grinding of the beads to function in a semi-continuous manner.

Advantageously, the beads may be agitated by ultrasound (generally leading to rather small DNA fragments of around 100 - 200 bp), by mechanical shaking or by mechanical vibration, preferably for approximately up to ten or so minutes. Even more advantageously, according to the present invention, the beads are agitated by mechanical shaking or vibration, using for example a rotating setup, generally leading to a size of DNA fragments of between 200 - 2000 kB. The increase in temperature created during the agitation and collision of the particles also appears to contribute to the effectiveness of the breaking open of the cells. The addition of chemical agents or of enzymes, such as proteases, may also promote the splitting open of the cells, in particular by digesting the cell walls.

Advantageously, during the breaking open of the cells, or subsequent to the breaking open of the cells and prior to the concentrating of DNA and/or of RNA, at least one chaotropic agent, advantageously a guanidine salt, such as guanidine thiocyanate, and at least one adsorption-modifying agent, advantageously an alcohol such as ethanol, may be added to the reaction medium containing the cells, preferably with the aid of pumps and valves, in order to facilitate the extraction of DNA and/or of RNA from the cells and in order to inactivate the nucleases, which are enzymes capable of destroying the nucleic acids. The guanidine salt, such as guanidine thiocyanate, is preferably added, according to the present invention, at a concentration of more than one mole per litre. Even more advantageously, a mixture of chaotropic agent and adsorption-modifying agent may be added to the reaction medium containing the cells from the pathogenic organisms.

Advantageously, the step for concentrating DNA and/or RNA, which follows that for breaking open the cells, may be carried out by reversible adsorption of the nucleic acids onto a solid support containing an adsorbent material, followed by elution of these acids.

Care is necessary to avoid trapping of nucleic acids at the walls of the system by strong or irreversible adsorption. Especially metal surfaces such as stainless steel tubes, rings or containers have a high tendency to trap the nucleic acids and are thus advantageously avoided. Thus, all adsorptive metal parts are advantageously avoided or eluted after DNA contact.

According to the present invention, the step for concentrating DNA and/or RNA corresponds to the extraction and purification of DNA and/or RNA.

The adsorbent material is advantageously a vitreous material, preferably comprising surfaced hydroxy groups, or a silica-based compound.

The elution of the nucleic acids is advantageously carried out by washing the absorbent material using at least one solvent containing monovalent ions, advantageously chosen from the group consisting of sodium thiocyanate, potassium thiocyanate and EDTA. The solvent according to the invention is thus advantageously a complexing agent made of monovalent ions.

The Applicant has thus discovered, surprisingly, that the elution of the nucleic acids from the adsorption support cannot be carried out without the prior conversion of the complex of nucleic acids and of divalent ions, formed during the first step of concentrating the organisms, into a complex consisting of nucleic acids and of monovalent ions. Specifically, the complex of nucleic acids and of divalent ions is strongly attached to the solid adsorption support and cannot be desorbed and then eluted by simply washing using a solvent. The desorption, followed by the elution, of the nucleic acids is only made possible by adding a complexing agent made of monovalent ions. Such an agent thus makes it possible to exchange the divalent ions, attached to the nucleic acids, against monovalent ions and thus to form a new complex which may be desorbed much more easily than the nucleic acid/divalent ions complex. After carrying out the ion exchange, a solvent is then added to finalize the elution of the nucleic acids. This solvent must be compatible with the amplification step which follows the step for concentrating the nucleic acids, and is advantageously chosen from the group consisting of water or a diluted buffer.

According to the present invention, the process may also contain a step for concentrating the volume in order to attain volumes of about one hundred µl up to less than 1 ml, advantageously less than 100 µl, before carrying out the nucleic acid amplification and subsequent to the step for concentrating the nucleic acids.

The step for detecting the pathogenic organisms, which follows that of nucleic acid amplification, may be on-line (real-time), in situ, detection. The step for detecting the pathogenic organisms is preferably carried out by fluorescence, by luminescence or by mass spectrometry. It may also be carried out by other detection techniques well known to those skilled in the art, such as those of hybridization on chips or of enzymatic amplification.

The detection may be carried out in situ by fluorescence. In order to obtain a detection signal, CyberGreen or another double-stranded intercalation fluorophore may thus be added during the amplification step, making it possible to indicate the success of the amplification by a strong increase in fluorescence during the cycle. Specifically, the primers labelled using the fluorophores hybridize specifically with the target DNA of the sample. Since the PCR amplification reaction generates a large number of copies of the target DNA, it is thus possible to obtain a quantification of the intensity of the fluorescence signal, which is directly correlated with the initial amount of target DNA in the sample analysed.

In order to obtain multispecific detection, i.e. detection specific for several pathogenic agents, in a single vial and in order to increase the specificity with respect to these pathogenic agents if this is required, molecular beacons or similar tracers can be added to the various vials used during the amplification. Using standard instrumentation, it is possible to detect up to four different species per vial.

The detection process may also contain a prior filtration step, before carrying out the step for concentrating the pathogenic organisms. Such a filtration may prove to be useful when analysing samples of crude water or of surface water, particularly samples contaminated and loaded with troublesome substances such as sand.

The detection process may also contain a prior step for treating the water sample by heat shock and/or by a nutrition stress, with for example glycerol or ethanol as carbon source, and/or by adding a gene-inducing chemical compound in order to induce the RNA of specific genes and to detect the viable pathogenic organisms in said water sample. Specifically, a heat shock (an increase in the temperature for a few moments) or the addition of a chemical compound induces a set of proteins thus allowing an RT (reverse transcriptase) reaction to be carried out as a first step, when the first PCR is carried out, which thus makes it possible to obtain a signal specific for viable organisms. The RT reaction allows the transformation of RNA into DNA. If it proves to be necessary, DNAases can be added in order to destroy the native DNA. Specificity is obtained by inducing a DNA which is specific to a high degree.

A heat shock may be performed by heat pulse for less than 3 hours, advantageously at a temperature of 30 to 50°C.

A gene-inducing chemical compound may be added. Such a compound makes it possible to induce the RNA of specific genes. The compound is advantageously a nutrient element, such as lactose, or a targeted inducer, such as IPTG (Isopropyl-(beta)-D-thiogalactopyranoside). This chemical compound allows a large increase in the level of specific RNAs in the cells. The duration of the induction depends on the organism, but is typically approximately between 2 minutes and 1 hour. The heat-induced proteins, such as dnaJ, grpE, hscB, hslJ, hslV, htpG, htpX, htrB, htrC, pphA, pphB, rpoE, degP, groEL, clpA, dnaK, inpA/B and other heat-shock proteins (for example of the small HSPs, HSP 60, HSP 70, HSP 90, HSP 100 and ubiquitin classes), are effective targets.

Inducing the RNA of specific genes can also be carried out by UV-irradiation, by using toxic substances as e.g. cis-platinum, anisomycin, tunicamycin, arsenites, by osmotic shock, by ethanol or by a minimal medium with for example glycerol as carbon source. For the purposes of the present invention, the expression "minimal medium" is intended to mean a medium which contains the minimal amount of chemicals needed for the organisms to survive.

The induction is direct proof of the viability of the cell. Furthermore, RNA can thus be detected in the form of many copies with greater sensitivity compared to the DNA. Although the detection of DNA is important, since the DNA is present in each cell in a fixed amount and it is, therefore, easy to make a correlation between the DNA and the number of cells, the RNA can, itself, be induced at various levels, thus making it possible to detect the viability of the pathogenic organisms.

Advantageously, the gene-inducing chemical compound may be lactose or IPTG so as to induce the lac Z RNA. The lac Z operon, which has a unique sequence in a certain number of pathogenic organisms, in fact constitutes a useful target. The duration of induction is approximately a few minutes.

The detection of the viable pathogenic organisms in a water sample may be carried out after having obtained a DNA signal representative of the presence of pathogenic organisms in said water sample. Thus, all of the sequential steps of the process for detecting the presence of pathogenic organisms may be first carried out in order to conclude on the possible contamination of the water sample analysed. Then, if a DNA signal is obtained, all of the sequential steps of the process are again carried out but a prior step for treating the water sample by heat shock and/or by adding a gene-inducing chemical compound is added in order to induce the RNA of specific genes. The DNA signal is then compared to the RNA signal and reliable viability data can then be obtained.

Advantageously, the control logic system consists of at least one computer, for instance of the PC type, which controls all of the steps. The control logic system thus makes it possible to control the various steps of the process and, at the end of the process, to receive the analytical data relating to the detection of the organisms.

Advantageously, the analytical data relating to the detection of the organisms are automatically transferred to a central control system. Advantageously, this central control system consists of at least one computer. This central control system will be able to group together, analyse and control all of the analytical data, for example using databases, and incorporate these data into mathematical models which will make it possible to predict the level of contamination of the water networks analysed, the type of microorganism present in these networks and also the survival rate of these microorganisms.

Advantageously, the process can function continuously and autonomously for a period of time greater than or equal to one day, preferably from three days to seven days. For example, this period of time depends on the number of reagents used. In addition, a given water sample can be assayed and analysed several time a day using the detection process according to the present invention. To do this, specific DNA sequences (primers) are used and, if necessary, new primers can be developed. The assay duration for a test cycle using the process according to the present invention is optimized at a period of time which is on the order of the doubling time of most organisms (2 to 6 hours).

A subject of the present invention is also a device as defined in claim 19, for detecting the presence of pathogenic organisms in a water sample.

Advantageously, the unit for amplifying the nucleic acids (4) may consist of at least one thermocycler polymerase chain reaction machine. The PCR machine carries out sequential amplification cycles, each cycle being carried out at a given range of temperature and for a given period of time. If necessary, according to the present invention, the amplification unit consists of two thermocycler polymerase chain reaction machines in order to obtain maximum selectivity with respect to the pathogenic agents whose detection in the water is being sought. The two machines are then used sequentially. The transfer of the nucleic acids from one machine to the other is carried out using automatic sampling and distribution means (8) and a control logic system (6) which controls these means.

A thermocycler machine with integrated on-line detection may be used to detect the amplification of the specific DNA. This type of machine thus consists of a thermocycler PCR machine with an integrated on-line detection chamber. When carrying out a nested PCR, the second PCR is carried out in this type of machine, the first PCR being carried out in a standard thermocycler polymerase chain reaction machine. Such a machine can be acquired from certain companies such as Biorad, Cepheid, Perkin Elmer or Roche Molecular Biochemicals. In this particular embodiment of the present invention, amplification unit (4) and detection unit (5) are grouped together in the same machine.

Advantageously, the unit for concentrating the pathogenic organisms (1) is a reactor made of metal, comprising a system for introducing the water sample, advantageously consisting of valves, a system for introducing various reagents, advantageously consisting of tubes, of valves and of rotary or cam-operated pumps, an agitation system, a system for evacuating the supernatant liquid, advantageously consisting of tubes and of valves, a system for evacuating the precipitate, advantageously consisting of valves and of pumps and advantageously placed at the bottom of the reactor, and a heating system advantageously consisting of a heating jacket (sheath) placed around the reactor.

The reactor is made of metal, advantageously of stainless steel, in order to obtain a high resistance to the chemical reagents introduced into said reactor. The reactor advantageously has a volume of 100 ml to 10 litres, preferably from 1 to 3 litres. The tubes are, themselves, advantageously made of steel or of plastic.

The introduction of the water sample according to the present invention is carried out automatically via valves which are opened and closed with the aid of the control logic system (6). The introduction of the various reagents required for concentrating the pathogenic organisms is carried out using tubes, valves and rotary or cam-operated pumps, said valves and pumps also being activated by the control logic system (6). The agitation and the heating are also controlled by the control logic system (6). The internal temperature of the reactor is advantageously controlled using sensors.

Advantageously, the unit for breaking open the cells (2) contains a bead grinder. Advantageously, the beads used are chemically inert and should be sufficiently solid to cause the cells to rupture. The beads used according to the present invention are advantageously small beads, such as polymer beads, for instance plastic, the diameter of which can range from a few mm to a few tens of µm. In order to improve the splitting open of the cells from the pathogenic organisms, the beads are agitated by a strong source of energy. The possible sources of energy for agitating the beads can be ultrasound sources, shakers of the rotary shaker type, devices of the Ultradurax type, devices of the sonotrode type or forceful vibrators.

Advantageously, the unit for concentrating DNA and/or RNA (3) contains one or more solid support(s) containing a reversible adsorption material for nucleic acids, advantageously a vitreous paste. The nucleic acids can thus be concentrated, for example using a porous absorption column containing a vitreous material, preferably comprising surfaced hydroxy groups, or a silica-based material, which will allow the reversible adsorption of the nucleic acids to the solid support.

Even more advantageously, the solid supports consist of microtitration plates containing multiwells, advantageously 60-, 96- or 384-well plates which, themselves, contain adsorbent materials. Each well corresponds to a reversible adsorption microcolumn.

Even more advantageously, the wells of the microtitration plates are used only once and are moved automatically in order to receive the liquid sample originating from the unit for breaking open the cells from the organisms. Compressed air may be injected in order to carry the fluid into the wells. The plate is referenced with respect to a system of XY coordinates, X corresponding to the axis along the width of the plate and Y corresponding to the axis along the length of the plate. The automatic movement of the plate, and consequently that of the wells which are attached to the wells, takes place along the X and Y axes, for example using the arm of an automaton. The automatic movement can be activated by the control logic system according to the present invention. In a particular embodiment of the present invention, the automatic movement is carried out using an automaton, which is controlled using a control logic system (6) advantageously consisting of a computer. Advantageously, the automaton has an arm which can move around according to an XYZ frame of reference.

Advantageously, the wells of the microtitration plates (or tube racks) can be protected against evaporation with a drop of fluid having a low specific density with a transparent lid.

After elution of the various nucleic acid samples present in the wells used during the adsorption, all the liquid samples are grouped together in order to be able to carry out the subsequent steps for amplifying the nucleic acids and for detecting the pathogenic organisms.

Advantageously, the detection unit (5) consists of a fluorescence detector. Advantageously, the fluorescence detector comprises an optical unit such as an electronic camera which is able to record the photons coming from the fluorophores and to convert them into electrons. Subsequently, the electronic picture is read out and transferred to the control logic system (6).

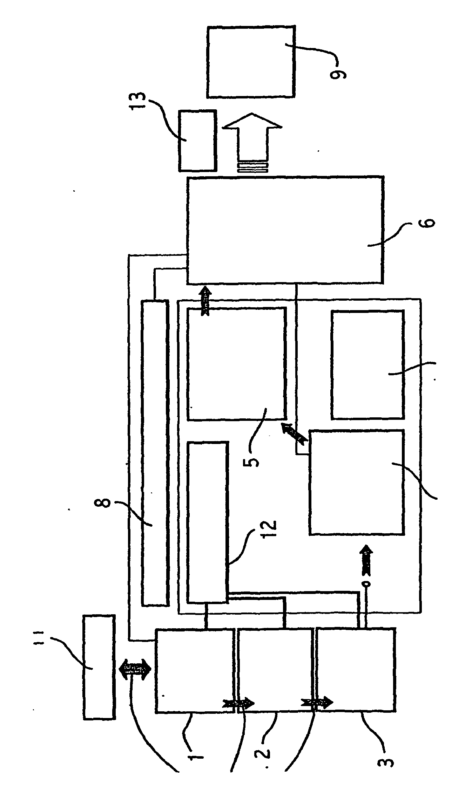

The functioning of the device according to the invention will be more clearly understood upon reading the description given hereinafter with reference to the attached Figure 1, which diagrammatically illustrates a particular embodiment of the present invention.

The detection device according to the present invention comprises at least one unit for concentrating the pathogenic organisms (1), a unit for breaking open the cells from the pathogenic organisms (2), a unit for concentrating (adsorbing then eluting) the nucleic acids (3), a unit for amplifying the nucleic acids (4), a unit for detecting the nucleic acids (5), a control logic system (6) consisting of a computer of PC type which controls the entire device, pumping means (7), an automatic sampling and distribution means (8) consisting of an automaton, a central control system (9) interfaced with a network (13) which makes it possible to transfer the data from the control logic system (6) to the central control system (9) consisting of a computer of PC type, a cooling area (10), an inlet and outlet system for the water sample (11) and an area (12) for storing the reagents required for units (1), (2), (3), (4) and (5).

Advantageously, the samples containing the pathogenic organisms circulates from the unit for concentrating the pathogenic organisms (1) to the inlet of the unit for amplifying the nucleic acids (4), via the units for breaking open the cells (2) and for concentrating the nucleic acids (3), using pumping means (7) advantageously consisting of tubes equipped with valves and/or pumps. Thus, the pumping means (7) form a fluidic system which is able to connect the units (1), (2), (3) and (4). The pumping means (7) according to the invention are activated and controlled by the control logic system (6).

Advantageously, according to the present invention, after the DNA and/or RNA has/have been concentrated, the nucleic acids are manipulated, from the inlet of the unit for amplifying the nucleic acids (4) to the outlet of the unit for detecting the nucleic acids (5), using automatic sampling and distribution means (8) advantageously consisting of an automaton. Thus, the units (4) and (5) are not directly connected to each other (no fluidic system). The automatic sampling and distribution means according to the invention are activated and controlled by the control logic system (6).

Advantageously, the control logic system (6) may consist of at least one computer, advantageously of the PC type, which manages and controls the entire device and which receives, controls and processes all of the analytical data relating to the detection of the pathogenic organisms.

Advantageously, the control logic system (6) is interfaced with a network for transferring, automatically or on demand, the analytical data relating to the detection of the organisms to a central control system (9).

Advantageously, the central control system (9) may consist of at least one computer. This central control system (9) is able to get and record analytical data, and then to analyse and to control them.

Advantageously, the device may also contain a cooling area (10) for storing the sensitive reagents, preferably a cooling block, which is protected against the condensation of the steam.

Advantageously, the device may be directly connected to the drinking water supply network for automatic sampling. The detection device according to the present invention thus makes it possible to test, in an entirely automated way, samples taken from different supply networks, such as the drinking water network. It can also be reused many times without needing to change the various units used.

Typically, the succession of the various steps of the detection process can be advantageously carried out as follows:
1. The unit (1) for concentrating the pathogenic organisms is flushed, cleaned and then filled with a volume of 100 ml to 5 litres of water, preferably of 2 to 3 litres of water. The water is introduced into the reactor using valves, the opening of which is activated by a computer (6).
2. Various reagents, such as sodium carbonate and calcium acetate, are added to the reactor via valves and rotary pumps, which are controlled by the computer (6). An inclusion precipitate is then formed at the bottom of the chamber, to which the cells and the nucleic acids from the pathogenic organisms adsorb.
3. The supernatant liquid is released, by example by gravity, using a tube and a valve.
4. The precipitate is dissolved using an acid, added to the reactor via a pump which is activated by the computer (6). A buffer agent is also added in the same way.
5. The solution containing the pathogenic organisms is transferred into the unit (2) for breaking open the cells from the organisms, which consists of a bead grinder, by gravity or through the action of a pump controlled by the computer (6). At this stage, the volume of solution entering the bead grinder is from 10 to 100 ml, preferably 50 ml.
6. The cells are broken open by grinding on agitated beads, by means of mechanical energy, for example with a rotary or vibrating shaker or an ultrasound source.
7. The ruptured cells are then transferred, via a pump controlled by the computer (6), into a container made of plastic, of approximately 100 ml.
8. A chaotropic agent and an adsorption-modifying agent are added via valves and pumps which are controlled by the computer (6).
9. The DNA and/or the RNA from the cells is then adsorbed on a solid support, advantageously on microtitration plates containing wells, said wells, themselves, containing an adsorbent material such as a vitreous material. Compressed air may be injected in order to carry the fluid into the wells, and an XY or XYZ frame of reference is used to manipulate the matrix of adsorbent columns. The wells, which are each microcolumns, can thus be manipulated using an automaton activated by the computer (6), the arm of which automaton moves the plate containing the wells according to an XY or XYZ frame of reference.
10. A monovalent complexing agent is then added via valves and pumps controlled by the computer (6), in order to replace the divalent ions complexed with the nucleic acids, with monovalent ions. The nucleic acid/monovalent ion complex thus formed is therefore, at this stage, still attached to the adsorption columns.
11. The nucleic acids are then eluted by adding a buffer, such as TRIS/EDTA, or water using rotary pumps.
12. The nucleic acids are pooled in a tube manipulated, for example, by an automaton. From this stage, all manipulations are carried out using an automatic sampling and distribution means, such as an automaton (8).
13. Optionally, and if it proves to be necessary, the total volume of solution containing the various pooled and eluted nucleic acids is decreased in order to attain volumes of about one hundred or so µl to less than 1 ml, before carrying out the nucleic acid amplification or several amplifications performed in parallel.
14. The nucleic acids are transferred into a thermocycler machine using the automaton (8) and the first amplification is then carried out in the machine by adding, first of all, the reagents for performing the PCR, such as the PCR buffer, the set of primers 1, the TAQ and the nucleotides.
15. The mixture of nucleic acids amplified a first time is then separated into several vials in order to detect a number of organisms at maximum sensitivity with respect to the pathogenic agents whose detection is being sought.
16. A second amplification is then carried out in real time in a second thermocycler machine by adding, in particular, the set of primers 2 (TAQ and nucleotide). Visualization and detection agents, such as fluorescence agents, are also added. The detection agents will thus be able to hybridize to the amplified fragments from the microorganisms. All the sample manipulations (transfers, addition of the various reagents, etc.) are carried out using the automaton (8) mentioned above.
17. The fluorescence is detected in situ during the second amplification (on-line detection).
18. The analytical data relating to the detection of the pathogenic organisms are visualized on the computer (6) and transferred to a central control system (9). All of the sequential steps of the process are managed and controlled using a control logic system (6) consisting of a computer.

The following examples are given in a nonlimiting capacity and illustrate the present invention.

### Examples of implementation of the invention:

### Example 1: Precipitation by inclusion of pathogenic agents

First of all, a water sample, taken directly from the drinking water supply network, from the environment or originating from a laboratory or from another source, is received in a 2-litre reactor and is heated to 50°C.

10 ml of 1 M TRIS (buffer agent) are first added so as to attain a pH of 8.5, followed by 25 ml of 1 M calcium acetate. The two compounds are then mixed rapidly, and then the mixing is stopped.

10 ml of 1 M Na₂CO₃ are then added and the various compounds are allowed to react for 3 minutes. Mixing is then carried out carefully for approximately 5 seconds, followed by the addition of 10 ml of Na₂CO₃, and then reaction medium is allowed to react for a further 3 minutes. Mixing is carried out gently but sufficiently and the reaction medium is again allowed to react for a further 10 minutes. 7.5 ml of 1 M calcium acetate are added and allowed to react for 3 minutes. Mixing is carried out carefully, 10 ml of Na₂CO₃ are added and the mixture is again allowed to react for 5 minutes.

The supernatant is decanted by opening a valve. The precipitate is dissolved by adding 13 ml of 20% formic acid. The mixture is then buffered by adding 2 M TRIS base to increase the pH to 7.2.

### Example 2: Amplification by PCR and detection

2.1 A thermocycler machine with integrated on-line detection, such as the LightCycler machine sold by Roche Molecular Biochemicals, is used to detect the amplification of the specific DNA. 32 samples are manipulated in parallel, and 40 amplification cycles are carried out at high temperature (from 90°C to 100°C), the 40 cycles lasting approximately 45 minutes. The amplification and the detection take place in capillary tubes. Detection limit is about one pathogenic organism.
2.2 As an alternative, it is also possible to use the ABI prism 7700 detection system, sold by PE Biosystems, processing 96 samples in parallel (two hours), over 40 cycles for the detection of more than 10 samples in a vial, for approximately 2.5 hours.

Sensitivities of the order of a few femtogrammes of total DNA/RNA (which is equivalent to one single cell) are thus obtained. Detection limit is about one pathogenic organism.

## Claims

1. Process for detecting the presence of pathogenic organisms in a water sample, comprising the succession of the following steps:
concentration of the pathogenic organisms from a water sample, wherein the concentrating of the pathogenic organisms is carried out by precipitation, coprecipitation or precipitation by inclusion,
breaking open of the cells of the pathogenic organisms,
concentration of DNA and/or of RNA,
at least one nucleic acid amplification, and
a qualitative and/or quantitative detection of DNA and/or RNA representative of the water contamination by said pathogenic organisms; wherein
all of the sequential steps of the process are controlled fully automatically using a control logic system.

2. Detection process according to Claim 1, **characterized in that** the pathogenic organisms detected are bacteria, viruses, fungi and/or protozoa.

3. Detection process according to Claim 1 or 2, **characterized in that** the starting volume of the water sample is of more than 50 milliters.

4. Detection process according to any one of Claims 1 to 3, **characterized in that** the nucleic acid amplification is carried out by a real-time polymerase chain reaction.

5. Detection process according to any one of Claims 1 to 4, **characterized in that** the concentrating is carried out in the presence of divalent metal and carbonate ions advantageously chosen from the group consisting of calcium carbonate, strontium carbonate and barium carbonate.

6. Detection process according to any one of Claims 1 to 5, **characterized in that** the breaking open of the cells is carried out by grinding on agitated beads.

7. Detection process according to Claim 6, **characterized in that** the beads are agitated by ultrasound, by mechanical shaking or by mechanical vibration.

8. Detection process according to any one of the preceding claims, **characterized in that**, during the breaking open, or subsequent to the breaking open of the cells and prior to the concentrating of DNA and/or of RNA, at least one chaotropic agent, advantageously a guanidine salt, such as guanidine thiocyanate, and at least one adsorption-modifying agent, advantageously an alcohol such as ethanol, are added to the reaction medium containing the cells.

9. Detection process according to any one of the preceding claims, **characterized in that** the concentrating of DNA and/or of RNA is carried out by reversible adsorption of the nucleic acids onto a solid support containing an adsorbent material, followed by elution of these acids.

10. Detection process according to Claim 9, **characterized in that** the elution of the nucleic acids is carried out by washing the absorbent material using at least one solvent containing monovalent ions, advantageously chosen from the group consisting of sodium thiocyanate, potassium thiocyanate and EDTA.

11. Detection process according to any one of the preceding claims, **characterized in that** the detection of the pathogenic organisms is an on-line detection, advantageously carried out by fluorescence, by luminescence or by mass spectrometry.

12. Detection process according to any one of the preceding claims, **characterized in that** it also comprises a prior filtration step.

13. Detection process according to any one of the preceding claims, **characterized in that** it also comprises a prior step for treating the water sample by heat shock and/or by a nutrition stress and/or by adding a gene-inducing chemical compound in order to induce the RNA of specific genes and to detect the viable pathogenic organisms in said water sample.

14. Detection process according to Claim 13, **characterized in that** the heat shock is carried out by heat pulse for less than 3 hours.

15. Detection process according to Claim 13 or 14, **characterized in that** the gene-inducing chemical compound is lactose or IPTG so as to induce the lac Z RNA.

16. Detection process according to any one of the preceding claims, **characterized in that** the control logic system consists of a computer of the PC type.

17. Detection process according to any one of the preceding claims, **characterized in that** the analytical data relating to the detection of the organisms are automatically transferred to a central control system.

18. Detection process according to any one of the preceding claims, **characterized in that** the process can function continuously and autonomously for a period of time greater than or equal to one day, preferably from three days to more than seven days.

19. Device for detecting the presence of pathogenic organisms in a water sample, comprising:
a unit for concentrating the pathogenic organisms (1),
a unit for breaking open cells from the pathogenic organisms (2),
a unit for concentrating DNA and/or RNA (3),
a unit for amplifying nucleic acids (4), and
a unit for qualitatively and/or quantitatively detecting DNA and/or RNA (5) representative of the water contamination by said pathogenic organisms;
at least one a control logic system (6) which controls fully automatically the sequential treatment steps carried out by units (1) to (5),
pumping means (7),
automatic sampling and distribution means (8), and
a central control system (9);
**characterized in that** the unit for concentrating the pathogenic organisms is adapted for precipitation, coprecipitation or precipitation by inclusion.

20. Detection device according to Claim 19, **characterized in that** the unit for amplifying the nucleic acids (4) consists of at least one thermocycler polymerase chain reaction machine.

21. Detection device according to Claim 19 or 20, **characterized in that** the unit for concentrating the pathogenic organisms (1) is a reactor made of metal, comprising a system for introducing the water sample, a system for introducing various reagents, an agitation system, a system for evacuating the supernatant liquid, a system for evacuating the precipitate, advantageously placed at the bottom of the reactor, and a heating system advantageously consisting of a heating jacket placed around the reactor.

22. Detection device according to any one of Claims 19 to 21, **characterized in that** the unit for breaking open the cells (2) contains a bead grinder.

23. Detection device according to any one of Claims 19 to 22, **characterized in that** the unit for concentrating DNA and/or RNA (3) comprises one or more solid support(s) containing a reversible adsorption material for nucleic acids, advantageously a vitreous material.

24. Detection device according to Claim 23, **characterized in that** the solid supports consist of microtitration plates containing multiwells, advantageously 60-, 96- or 384-well plates.

25. Detection device according to Claim 24, **characterized in that** the wells of the microtitration plates are used only once and are moved automatically in order to receive the liquid sample originating from the unit for breaking open the cells from the organisms.

26. Detection device according to Claim 25, **characterized in that** the automatic movement of the wells is carried out using an automaton.

27. Detection device according to any one of Claims 19 to 26, **characterized in that** the detection unit (5) consists of a fluorescence detector, advantageously comprising an electronic camera.

28. Detection device according to any one of Claims 19 to 27, **characterized in that** the sample containing the pathogenic organisms circulates from the unit for concentrating the pathogenic organisms (1) to the inlet of the unit for amplifying the nucleic acids (4), via the units for breaking open the cells (2) and for concentrating the nucleic acids (3), using pumping means (7) advantageously consisting of tubes equipped with valves and/or pumps.

29. Detection device according to any one of Claims 19 to 28, **characterized in that** after the DNA and/or RNA has/have been concentrated, the nucleic acids are manipulated, from the inlet of the unit for amplifying the nucleic acids (4) to the outlet of the unit for detecting the nucleic acids (5), using automatic sampling and distribution means (8) advantageously consisting of an automaton.

30. Detection device according to any one of Claims 19 to 29, **characterized in that** the control logic system (6) consists of at least one computer of the PC type which controls the entire device.

31. Detection device according to any one of Claims 19 to 30, **characterized in that** the control logic system (6) is interfaced with a network for transferring, automatically or on demand, the analytical data relating to the detection of the organisms to a central control system (9).

32. Detection device according to any one of Claims 19 to 31, **characterized in that** the central control system (9) consists of at least one computer.

33. Detection device according to any one of Claims 19 to 32, **characterized in that** the device also contains a cooling area (10) for storing the sensitive reagents, preferably a cooling block.

34. Detection device according to any one of Claims 19 to 33, **characterized in that** the device is directly connected to the drinking water supply network for automatic sampling

## Patentansprüche

1. Verfahren zum Nachweis des Vorliegens pathogener Organismen in einer Wasserprobe, wobei das Verfahren die Abfolge der folgenden Schritte umfasst:
Konzentration der pathogenen Organismen aus einer Wasserprobe, wobei das Konzentrieren der pathogenen Organismen durch Präzipitation, Kopräzipitation oder Präzipitation durch Inklusion durchgeführt wird,
Aufbrechen der Zellen der pathogenen Organismen,
Konzentration von DNA und/oder RNA,
mindestens eine Nukleinsäurenamplifikation und
ein qualitativer und/oder quantitativer Nachweis von DNA und/oder RNA, die für die Wasserkontamination durch die besagten pathogenen Organismen repräsentativ ist;
wobei alle aufeinander folgenden Schritte des Verfahrens vollautomatisch unter Verwendung eines Steuerlogiksystems gesteuert werden.

2. Nachweisverfahren nach Anspruch 1, **dadurch gekennzeichnet, dass** die nachgewiesenen pathogenen Organismen Bakterien, Viren, Pilze und/oder Protozoen sind.

3. Nachweisverfahren nach Anspruch 1 oder 2, **dadurch gekennzeichnet, dass** das Ausgangsvolumen der Wasserprobe mehr als 50 Milliliter ist.

4. Nachweisverfahren nach einem der Ansprüche 1 bis 3, **dadurch gekennzeichnet, dass** die Nukleinsäurenamplifikation mittels einer Echtzeit-Polymerase-Kettenreaktion durchgeführt wird.

5. Nachweisverfahren nach einem der Ansprüche 1 bis 4, **dadurch gekennzeichnet, dass** das Konzentrieren in Gegenwart zweiwertiger Metall- und Carbonationen durchgeführt wird, die vorteilhafterweise aus der Gruppe bestehend aus Calciumcarbonat, Strontiumcarbonat und Bariumcarbonat ausgewählt sind.

6. Nachweisverfahren nach einem der Ansprüche 1 bis 5, **dadurch gekennzeichnet, dass** das Aufbrechen der Zellen durch Mahlen auf bewegten Kugeln durchgeführt wird.

7. Nachweisverfahren nach Anspruch 6, **dadurch gekennzeichnet, dass** die Kugeln durch Ultraschall, durch mechanisches Schütteln oder durch mechanische Vibration bewegt werden.

8. Nachweisverfahren nach einem der vorhergehenden Ansprüche, **dadurch gekennzeichnet, dass** während des Aufbrechens oder nach dem Aufbrechen der Zellen und vor dem Konzentrieren von DNA und/oder RNA mindestens ein chaotropes Mittel, vorteilhafterweise ein Guanidinsalz, wie Guanidinthiocyanat, und mindestens ein Adsorptionsmodifizierungsmittel, vorteilhafterweise ein Alkohol, wie Ethanol, dem Reaktionsmedium zugesetzt werden, das die Zellen enthält.

9. Nachweisverfahren nach einem der vorhergehenden Ansprüche, **dadurch gekennzeichnet, dass** das Konzentrieren von DNA und/oder RNA durch reversible Adsorption der Nukleinsäuren auf einem festen Träger, der ein Adsorbens enthält, gefolgt von Elution dieser Säuren durchgeführt wird.

10. Nachweisverfahren nach Anspruch 9, **dadurch gekennzeichnet, dass** die Elution der Nukleinsäuren durch Waschen des Adsorbens unter Verwendung mindestens eines Lösungsmittels, das einwertige Ionen enthält, die vorteilhafterweise aus der Gruppe bestehend aus Natriumthiocyanat, Kaliumthiocyanat und EDTA ausgewählt sind, durchgeführt wird.

11. Nachweisverfahren nach einem der vorhergehenden Ansprüche, **dadurch gekennzeichnet, dass** der Nachweis der pathogenen Organismen ein On-line-Nachweis ist, der vorteilhafterweise mittels Fluoreszenz, mittels Lumineszenz oder mittels Massenspektrometrie durchgeführt wird.

12. Nachweisverfahren nach einem der vorhergehenden Ansprüche, **dadurch gekennzeichnet, dass** es auch einen vorherigen Filtrationsschritt umfasst.

13. Nachweisverfahren nach einem der vorhergehenden Ansprüche, **dadurch gekennzeichnet, dass** es auch einen vorherigen Schritt zum Behandeln der Wasserprobe durch Hitzeschock und/oder durch einen Ernährungsstress und/oder durch Zusetzen einer geninduzierenden chemischen Verbindung umfasst, um die RNA spezifischer Gene zu induzieren und die lebensfähigen pathogenen Organismen in der Wasserprobe nachzuweisen.

14. Nachweisverfahren nach Anspruch 13, **dadurch gekennzeichnet, dass** der Hitzeschock mittels Hitzeimpuls für weniger als 3 Stunden durchgeführt wird.

15. Nachweisverfahren nach Anspruch 13 oder 14, **dadurch gekennzeichnet, dass** die geninduzierende chemische Verbindung Lactose oder IPTG ist, um die lacZ-RNA zu induzieren.

16. Nachweisverfahren nach einem der vorhergehenden Ansprüche, **dadurch gekennzeichnet, dass** das Steuerlogiksystem aus einem Computer des PC-Typs besteht.

17. Nachweisverfahren nach einem der vorhergehenden Ansprüche, **dadurch gekennzeichnet, dass** die analytischen Daten, die den Nachweis der Organismen betreffen, automatisch auf ein zentrales Steuersystem übertragen werden.

18. Nachweisverfahren nach einem der vorhergehenden Ansprüche, **dadurch gekennzeichnet, dass** das Verfahren kontinuierlich und autonom für einen Zeitraum von länger als oder gleich einem Tag, vorzugsweise von drei Tagen bis länger als sieben Tage arbeiten kann.

19. Vorrichtung zum Nachweis des Vorliegens pathogener Organismen in einer Wasserprobe, wobei die Vorrichtung Folgendes umfasst:
eine Einheit zum Konzentrieren der pathogenen Organismen (1),
eine Einheit zum Aufbrechen von Zellen von den pathogenen Organismen (2),
eine Einheit zum Konzentrieren von DNA und/oder RNA (3),
eine Einheit zum Amplifizieren von Nukleinsäuren (4) und
eine Einheit zum qualitativen und/oder quantitativen Nachweisen von DNA und/oder RNA (5), die für die Wasserkontamination durch die besagten pathogenen Organismen repräsentativ ist;
mindestens ein Steuerlogiksystem (6), das die von den Einheiten (1) bis (5) durchgeführten aufeinander folgenden Behandlungsschritte vollautomatisch steuert,
Pumpmittel (7),
automatische Probenahme- und -verteilungsmittel (8) und
ein zentrales Steuersystem (9);
**dadurch gekennzeichnet, dass** die Einheit zum Konzentrieren der pathogenen Organismen zur Präzipitation, Kopräzipitation oder Präzipitation durch Inklusion eingerichtet ist.

20. Nachweisvorrichtung nach Anspruch 19, **dadurch gekennzeichnet, dass** die Einheit zum Amplifizieren der Nukleinsäuren (4) aus mindestens einer Thermocycler-Polymerase-Kettenreaktionsmaschine besteht.

21. Nachweisvorrichtung nach Anspruch 19 oder 20, **dadurch gekennzeichnet, dass** die Einheit zum Konzentrieren der pathogenen Organismen (1) ein aus Metall hergestellter Reaktor ist, der ein System zum Einbringen der Wasserprobe, ein System zum Einbringen verschiedener Reagenzien, ein Bewegungssystem, ein System zum Absaugen der Überstandsflüssigkeit, ein System zum Absaugen des Präzipitats, das vorteilhafterweise am Boden des Reaktors angeordnet ist, und ein Heizsystem, das vorteilhafterweise aus einem Heizmantel besteht, der um den Reaktor angeordnet ist, umfasst.

22. Nachweisvorrichtung nach einem der Ansprüche 19 bis 21, **dadurch gekennzeichnet, dass** die Einheit zum Aufbrechen der Zellen (2) ein Kugelmahlwerk enthält.

23. Nachweisvorrichtung nach einem der Ansprüche 19 bis 22, **dadurch gekennzeichnet, dass** die Einheit zum Konzentrieren von DNA und/oder RNA (3) einen oder mehrere feste Träger umfasst, das bzw. die ein reversibles Adsorbens für Nukleinsäuren, vorteilhafterweise ein glasartiges Material enthält bzw. enthalten.

24. Nachweisvorrichtung nach Anspruch 23, **dadurch gekennzeichnet, dass** die festen Träger aus Mikrotitrationsplatten besteht, die Multiwellplatten, vorteilhafterweise 60-, 96- oder 384-Well-Platten enthalten.

25. Nachweisvorrichtung nach Anspruch 24, **dadurch gekennzeichnet, dass** die Wells der Mikrotitrationsplatten nur einmal verwendet und automatisch bewegt werden, um die flüssige Probe aufzunehmen, die von der Einheit zum Aufbrechen der Zellen von den Organismen stammt.

26. Nachweisvorrichtung nach Anspruch 25, **dadurch gekennzeichnet, dass** das automatische Bewegen der Wells unter Verwendung eines Automaten durchgeführt wird.

27. Nachweisvorrichtung nach einem der Ansprüche 19 bis 26, **dadurch gekennzeichnet, dass** die Nachweiseinheit (5) aus einem Fluoreszenzdetektor besteht, der vorteilhafterweise eine elektronische Kamera umfasst.

28. Nachweisvorrichtung nach einem der Ansprüche 19 bis 27, **dadurch gekennzeichnet, dass** die Probe, die die pathogenen Organismen enthält, von der Einheit zum Konzentrieren der pathogenen Organismen (1) über die Einheiten zum Aufbrechen der Zellen (2) und zum Konzentrieren der Nukleinsäuren (3) zu dem Einlass der Einheit zum Amplifizieren der Nukleinsäuren (4) unter Verwendung von Pumpmitteln (7), die vorteilhafterweise aus Schläuchen bestehen, die mit Ventilen und/oder Pumpen ausgestattet sind, zirkuliert.

29. Nachweisvorrichtung nach einem der Ansprüche 19 bis 28, **dadurch gekennzeichnet, dass**, nachdem die DNA und/oder RNA konzentriert wurde bzw. wurden, die Nukleinsäuren von dem Einlass der Einheit zum Amplifizieren der Nukleinsäuren (4) zu dem Auslass der Einheit zum Nachweisen der Nukleinsäuren (5) unter Verwendung automatischer Probenahme- und - verteilungsmittel (8), die vorzugsweise aus einem Automaten bestehen, verarbeitet werden.

30. Nachweisvorrichtung nach einem der Ansprüche 19 bis 29, **dadurch gekennzeichnet, dass** das Steuerlogiksystem (6) aus mindestens einem Computer des PC-Typs besteht, der die gesamte Vorrichtung steuert.

31. Nachweisvorrichtung nach einem der Ansprüche 19 bis 30, **dadurch gekennzeichnet, dass** das Steuerlogiksystem (6) mit einem Netz zum Übertragen, automatisch oder auf Anfrage, der analytischen Daten, die den Nachweis der Organismen betreffen, zu einem zentralen Steuersystem (9) verknüpft ist.

32. Nachweisvorrichtung nach einem der Ansprüche 19 bis 31, **dadurch gekennzeichnet, dass** das zentrale Steuersystem (9) aus mindestens einem Computer besteht.

33. Nachweisvorrichtung nach einem der Ansprüche 19 bis 32, **dadurch gekennzeichnet, dass** die Vorrichtung außerdem einen Kühlbereich (10) zum Lagern der empfindlichen Reagenzien, vorzugsweise einen Kühlblock enthält.

34. Nachweisvorrichtung nach einem der Ansprüche 19 bis 33, **dadurch gekennzeichnet, dass** die Vorrichtung zur automatischen Probenahme direkt mit dem Trinkwasserversorgungsnetz verbunden ist.

## Revendications

1. Procédé permettant de détecter la présence d'organismes pathogènes dans un échantillon d'eau, comprenant la succession des étapes suivantes :
concentration des organismes pathogènes à partir d'un échantillon d'eau, ladite concentration des organismes pathogènes étant effectuée par précipitation, coprécipitation ou précipitation par inclusion,
rupture afin d'ouvrir les cellules des organismes pathogènes,
concentration de l'ADN et/ou de l'ARN,
au moins une amplification d'acides nucléiques, et
une détection qualitative et/ou quantitative de l'ADN et/ou de l'ARN représentatif de la contamination de l'eau par lesdits organismes pathogènes ; dans lequel
toutes les étapes séquentielles du procédé sont contrôlées de façon totalement automatisée à l'aide d'un système de logique de commande.

2. Procédé de détection selon la revendication 1, **caractérisé en ce que** les organismes pathogènes détectés sont des bactéries, des virus, des champignons et/ou des protozoaires.

3. Procédé de détection selon la revendication 1 ou 2, **caractérisé en ce que** le volume de départ de l'échantillon d'eau est supérieur à 50 millilitres.

4. Procédé de détection selon l'une quelconque des revendications 1 ou 3, **caractérisé en ce que** l'amplification d'acides nucléiques est effectuée par une amplification en chaîne par polymérase en temps réel.

5. Procédé de détection selon l'une quelconque des revendications 1 ou 4, **caractérisé en ce que** la concentration est effectuée en présence de métal divalent et d'ions carbonate avantageusement sélectionnés dans le groupe constitué par le carbonate de calcium, le carbonate de strontium et le carbonate de baryum.

6. Procédé de détection selon l'une quelconque des revendications 1 ou 5, **caractérisé en ce que** la rupture afin d'ouvrir les cellules est effectuée par broyage sur grains agités.

7. Procédé de détection selon la revendication 6, **caractérisé en ce que** les grains sont agités par ultrason, par secouage mécanique ou par vibration mécanique.

8. Procédé de détection selon l'une quelconque des revendications précédentes, **caractérisé en ce que**, au cours de la rupture afin d'ouvrir les cellules, ou à la suite de la rupture afin d'ouvrir les cellules et avant la concentration de l'ADN et/ou de l'ARN, au moins un agent chaotropique, avantageusement un sel de guanidine, tel qu'un thiocyanate de guanidine, et au moins un agent modificateur d'adsorption, avantageusement un alcool tel que l'éthanol, sont ajoutés au milieu réactionnel contenant les cellules.

9. Procédé de détection selon l'une quelconque des revendications précédentes, **caractérisé en ce que** la concentration de l'ADN et/ou de l'ARN est effectuée par adsorption réversible des acides nucléiques sur un support solide contenant une substance adsorbante, suivie par une élution de ces acides.

10. Procédé de détection selon la revendication 9, **caractérisé en ce que** l'élution des acides nucléiques est effectuée par lavage de la substance adsorbante à l'aide d'au moins un solvant contenant des ions monovalents, avantageusement sélectionnés dans le groupe contenant le thiocyanate de sodium, le thiocyanate de potassium et l'EDTA.

11. Procédé de détection selon l'une quelconque des revendications précédentes, **caractérisé en ce que** la détection des organismes pathogènes est une détection en ligne, avantageusement effectuée par fluorescence, par luminescence ou par spectrométrie de masse.

12. Procédé de détection selon l'une quelconque des revendications précédentes, **caractérisé en ce qu'**il comprend également une étape de filtration préalable.

13. Procédé de détection selon l'une quelconque des revendications précédentes, **caractérisé en ce qu'**il comprend également une étape préalable permettant de traiter l'échantillon d'eau par choc thermique et/ou par une contrainte nutritionnelle et/ou en ajoutant un composé chimique inducteur de gène afin d'induire l'ARN de gènes spécifiques et de détecter les organismes pathogènes viables dans ledit échantillon d'eau.

14. Procédé de détection selon la revendication 13, **caractérisé en ce que** le choc thermique est effectué par impulsion thermique de moins de 3 heures.

15. Procédé de détection selon la revendication 13 ou 14, **caractérisé en ce que** le composé chimique inducteur de gène est le lactose ou l'IPTG de manière à induire l'ARN lacZ.

16. Procédé de détection selon l'une quelconque des revendications précédentes, **caractérisé en ce que** le système de logique de commande est constitué d'un ordinateur de type PC.

17. Procédé de détection selon l'une quelconque des revendications précédentes, **caractérisé en ce que** les données analytiques concernant la détection des organismes sont automatiquement transférées à un système de commande centrale.

18. Procédé de détection selon l'une quelconque des revendications précédentes, **caractérisé en ce que** le procédé peut fonctionner en continu et de façon autonome pendant une durée supérieure ou égale à un jour, de préférence de trois jours à plus de sept jours.

19. Dispositif permettant de détecter la présence d'organismes pathogènes dans un échantillon d'eau, comprenant :
une unité de concentration des organismes pathogènes (1),
une unité de rupture afin d'ouvrir les cellules des organismes pathogènes (2),
une unité de concentration d'ADN et/ou d'ARN (3),
une unité d'amplification des acides nucléiques (4), et
une unité de détection de façon qualitative et/ou quantitative de l'ADN et/ou de l'ARN (5) représentatif de la contamination de l'eau par lesdits organismes pathogènes ;
au moins un système de logique de commande (6) qui commande de façon totalement automatique les étapes séquentielles du traitement effectuées par les unités (1) à (5),
un moyen de pompage (7),
un moyen d'échantillonnage et de distribution automatique (8), et
un système de commande centrale (9) ;
**caractérisé en ce que** l'unité de concentration des organismes pathogènes est adaptée à la précipitation, à la coprécipitation ou à la précipitation par inclusion.

20. Dispositif de détection selon la revendication 19, **caractérisé en ce que** l'unité d'amplification des acides nucléiques (4) est constituée au moins d'une machine d'amplification en chaîne par polymérase de cycleur thermique.

21. Dispositif de détection selon la revendication 19 ou 20, **caractérisé en ce que** l'unité de concentration des organismes pathogènes (1) est un réacteur constitué de métal, comprenant un système d'introduction de l'échantillon d'eau, un système d'introduction de divers réactifs, un système d'agitation, un système d'évacuation du liquide surnageant, un système d'évacuation du précipité, avantageusement placé au fond du réacteur, et un système de chauffage avantageusement constitué d'une enveloppe chauffante placée autour du réacteur.

22. Dispositif de détection selon l'une quelconque des revendications 19 à 21, **caractérisé en ce que** l'unité de rupture afin d'ouvrir les cellules (2) contient un broyeur à grains.

23. Dispositif de détection selon l'une quelconque des revendications 19 à 22, **caractérisé en ce que** l'unité de concentration de l'ADN et/ou de l'ARN (3) comprend un ou plusieurs support(s) solide(s) contenant une substance à adsorption réversible pour acides nucléiques, avantageusement une substance vitreuse.

24. Dispositif de détection selon la revendication 23, **caractérisé en ce que** les supports solides sont constitués de plaques de microtitration contenant plusieurs puits, avantageusement des plaques de 60, 96 ou 384 puits.

25. Dispositif de détection selon la revendication 24, **caractérisé en ce que** les puits des plaques de microtitration ne sont utilisés qu'une seule fois et sont déplacés automatiquement afin de recevoir l'échantillon liquide provenant de l'unité de rupture afin d'ouvrir les cellules des organismes.

26. Dispositif de détection selon la revendication 25, **caractérisé en ce que** le mouvement automatique des puits est effectué à l'aide d'un automate.

27. Dispositif de détection selon l'une quelconque des revendications 19 à 26, **caractérisé en ce que** l'unité de détection (5) est constituée d'un détecteur fluorimétrique, comprenant avantageusement une caméra électronique.

28. Dispositif de détection selon l'une quelconque des revendications 19 à 27, **caractérisé en ce que** l'échantillon contenant les organismes pathogènes circule depuis l'unité de concentration des organismes pathogènes (1) jusqu'à l'orifice d'entrée de l'unité d'amplification des acides nucléiques (4), en passant par l'unité de rupture afin d'ouvrir les cellules (2) et l'unité de concentration des acides nucléiques (3), à l'aide du moyen de pompage (7) avantageusement constitué de tubes équipés de soupapes et/ou de pompes.

29. Dispositif de détection selon l'une quelconque des revendications 19 à 28, **caractérisé en ce qu'**après la concentration de l'ADN et/ou de l'ARN, les acides nucléiques sont manipulés, depuis l'orifice d'entrée de l'unité d'amplification des acides nucléiques (4) jusqu'à l'orifice de sortie de l'unité de détection des acides nucléiques (5), à l'aide du moyen d'échantillonnage et de distribution automatique (8) avantageusement constitué d'un automate.

30. Dispositif de détection selon l'une quelconque des revendications 19 à 29, **caractérisé en ce que** le système de logique de commande (6) est constitué au moins d'un ordinateur de type PC qui commande la totalité du dispositif.

31. Dispositif de détection selon l'une quelconque des revendications 19 à 30, **caractérisé en ce que** le système de logique de commande (6) est interfacé avec un réseau permettant de transférer, automatiquement ou sur demande, les données analytiques concernant la détection des organismes vers un système de commande centrale (9).

32. Dispositif de détection selon l'une quelconque des revendications 19 à 31, **caractérisé en ce que** le système de commande centrale (9) est constitué au moins d'un ordinateur.

33. Dispositif de détection selon l'une quelconque des revendications 19 à 32, **caractérisé en ce que** le dispositif contient également une zone de refroidissement (10) pour stocker les réactifs sensibles, de préférence un bloc de refroidissement.

34. Dispositif de détection selon l'une quelconque des revendications 19 à 33, **caractérisé en ce que** le dispositif est directement connecté au réseau de distribution d'eau potable pour un échantillonnage automatique.
